# EUROPEAN PATENT SPECIFICATION

(11) **EP 0 312 996 B1**
(45) Date of publication and mention of the grant of the patent: **13.04.1994**
(21) Application number: 88117349.6
(22) Date of filing: 18.10.1988
(51) Int. Cl.: C12P 21/00, C12N 5/00, A61K 39/395, C12N 15/00, G01N 33/577

(54) **Anti-human bcdf monoclonal antibody**
Monoklonaler Antikörper gegen humanen BCDF
Anticorps monoclonal contre le bcdf humain

(30) Priority: 19.10.1987 JP 263631/87; 09.03.1988 JP 53828/88
(43) Date of publication of application: 26.04.1989
(73) Proprietor: Kishimoto, Tadamitsu, Prof., Tondabayashi Osaka-fu (JP)
(72) Inventor: Kishimoto, Tadamitsu, Tondabayashi Osaka-fu (JP); Hirano, Toshio, Ibaragi-shi Osaka-fu (JP)
(74) Representative: Strehl Schübel-Hopf Groening & Partner

(56) References cited:
- CHEMICAL ABSTRACTS, vol. 109, 1988, page 546, abstract no. 71738b, Columbus, Ohio, US; T. MATSUDA et al.: "Establishment of an interleukin 6 (IL 6)/B cell stimulatory factor 2-dependent cell line and preparation of anti-IL6 monoclonal antibodies" & EUR.J. IMMUNOL 1988, 18(6), 951-6
- BIOLOGICAL ABSTRACTS/RMM, vol. 28, 1985, no. 65431; L.K.L. JUNG et al.: "Selective inhibition of growth factor-dependent human B cell proliferation by monoclonal antibody AB-1 to an antigen expressed by activated B cells", & JOURNAL OF EXPERIMENTAL MEDICINE (US) 1984 (RECD. 1985) vol. 160, no. 6, p1919-1924
- BIOLOGICAL ABSTRACTS, vol. 84, no. 12, 1987, abstract no. 118862, Philadelphia, PA, US; K. YASUKAWA et al.: "Structure and expression of human B cell stimulatory factor-2 (BSF-2/IL-6) gene", & EMBO (EUR.MOL.BIOL.ORGAN.) J 6(10): 2939-2946, 1987
- Proc. Natl Acad. Sci., USA (1986), 83, 8957-8961, May, L.T. et al.
- EMBO J., (1986), 5 (10), 2529-2537, Zilberstein A. et al.

## Description

The present invention relates to a monoclonal antibody specific to human B cell differentiation factor or short "BCDF" and a hybridoma capable of producing the monoclonal antibody as well as an agent for treating autoimmune diseases such as rheumatoid arthritis or an anti-cancer agent containing as an effective ingredient a monoclonal antibody specific to human BCDF.

Human BCDF is a humoral factor produced by immune cells, a so called cytokine and is also known as human B cell stimulatory factor 2 (BSF-2) or interleukin 6 (IL-6). As shown below, the entire amino acid sequence of this substance has already been determined (Nature, 324, 73, 1986). The amino acid sequence is as follows: 
PRO VAL PRO PRO GLY GLU ASP SER LYS ASP VAL
ALA ALA PRO HIS ARG GLN PRO LEU THR SER SER
GLU ARG ILE ASP LYS GLN ILE ARG TYR ILE LEU
ASP GLY ILE SER ALA LEU ARG LYS GLU THR CYS
ASN LYS SER ASN MET CYS GLU SER SER LYS GLU
ALA LEU ALA GLU ASN ASN LEU ASN LEU PRO LYS
MET ALA GLU LYS ASP GLY CYS PHE GLN SER GLY
PHE ASN GLU GLU THR CYS LEU VAL LYS ILE ILE
THR GLY LEU LEU GLU PHE GLU VAL TYR LEU GLU
TYR LEU GLN ASN ARG PHE GLU SER SER GLU GLU
 
GLN ALA ARG ALA VAL GLN MET SER THR LYS VAL
LEU ILE GLN PHE LEU GLN LYS LYS ALA LYS ASN
LEU ASP ALA ILE THR THR PRO ASP PRO THR THR
ASN ALA SER LEU LEU THR LYS LEU GLN ALA GLN
ASN GLN TRP LEU GLN ASP MET THR THR HIS LEU
ILE LEU ARG SER PHE LYS GLU PHE LEU GLN SER
SER LEU ARG ALA LEU ARG GLN MET
Methods for the construction of this human BCDF have already been established. Known are, for example, a method for producing human BCDF which comprises culturing human T cell line VT-1 (IFO 50096) and a method for producing human BCDF which comprises inserting a human BCDF gene into an expression plasmid vector to transform the gene into host Escherichia coli and then culturing the transformant. Furthermore, the following method for production has been developed recently. A part of human interleukin 2 (hereafter simply referred to as IL2) gene is ligated with a human BCDF gene, the ligation product is then inserted into an expression plasmid vector and the plasmid is transfected into Escherichia coli. By culturing the thus obtained transformant, a fused protein having a part of the IL2 molecule (corresponding to 1-21 amino acid sequence) conjugated with human BCDF via Phe-Arg-Ala, namely, human IL-2-BCDF, can be produced in large quantities. By treating this fused protein with protease (kallikrein, if necessary, in combination with aminopeptidase -P) and purifying by reversed phase HPLC, human BCDF can be obtained in large quantities in an industrial scale (Japanese Patent Application No. 61-302699).

It is known that human BCDF shows various physiological activities such as inducing antibody production from activated B cells. On the other hand, it has been found that human BCDF is abnormally produced in a patient with cardiac myxoma or a patient with rheumatoid arthritis (JIKKEN IGAKU (Experimental Medicine), 5 , 816, 1987). From these findings, it is considered that human BCDF certainly plays an important role as a regulator of immune response. It is thus assumed that an assay for the concentration of human BCDF in blood, urine and body fluids from a patient with an autoimmune disease, cancer, immunodeficiency, etc. would provide an extrememly useful immune parameter in determining the abnormality of immune mechanism of the patient.

It is however, not known and not possible to directly measure the concentration of human BCDF quantitatively in blood, urine or body fluids from the patient. This is because only a trace amount of human BCDF is present and various contaminants which are contained in blood, urine and body fluids activate or interfere with the bioassay system of human BCDF.

On the other hand, hybridomas of spleen cells or lymph node cells with myeloma cells are described in several publications, for example, Koehler et al., Nature, 256, 495 (1975) and Eur. J. Immunol., 6, 511 (1976), Milstein et al., Nature, 266, 550 (1977); Walsh, Nature, 266 , 495 (1977). Therefore, it is considered that by producing a monoclonal antibody to human BCDF, namely, anti-human BCDF monoclonal antibody, using a hybridoma technique and by performing the radioimmunoassay (RIA) or enzyme-linked immunoassay (EIA) using anti-human BCDF monoclonal antibody thus obtained, a trace amount of human BCDF in a sample containing various contaminants could be determined.

It is furthermore considered that in synovial fluids and tissues of affected joints of rheumatoid arthritis patients, the cells would produce human BCDF and this human BCDF would induce the production of an autoantibody. In such a synovial tissue, human BCDF molecules are present within the cells. Therefore, by using anti-human BCDF monoclonal antibody, intracytoplasmic BCDF in a biopsy sample such as a tissue slice or tissue fluids, etc, can be immunohistochemically stained.

In addition, anti-human BCDF monoclonal antibody can also be used to purify human BCDF.
Furthermore, if anti-human BCDF monoclonal antibody capable of neutralizing human BCDF activity is obtained, such an antibody can also be used as a drug for treating autoimmune diseases such as rheumatoid arthritis when human BCDF is excessively produced, as described above. Anti-human BCDF monoclonal antibody capable of neutralizing human BCDF activity can also be used for treating cancer such as myeloma and plasmacytoma responsive to human BCDF to proliferate.

The EMBO Journal 1986, 5 (10) 2529-2537, discloses a study about the structure and the expression of a cDNA and genes for hIFNβ₂. In the course of this study a rabbit antiserum has been obtained which binds to hIFNβ₂, but not to hIFNβ₁.

However, before the present invention, it was quite unknown to establish a hybridoma from anti-human BCDF antibody-producing cells by using human BCDF as an antigen and to produce therefrom a monoclonal antibody to human BCDF,namely, anti-human BCDF monoclonal antibody.

Therefore, an object of the present invention is to provide a monoclonal antibody showing specificity to human BCDF and a hybridoma capable of producing this monoclonal antibody. It is further an object of the present invention to provide a drug for the treatment of autoimmune diseases or an anti-cancer agent containing the monoclonal antibody as an active ingredient.

As a result of extensive investigations to solve the foregoing problem, the present inventors could obtain anti-human BCDF monoclonal antibody by using human BCDF as an antigen and establish hybridomas capable of producing the monoclonal antibody.

According to the present invention, hybridoma clones capable of producing a monoclonal antibody showing specificity to human BCDF and several anti-human BCDF monoclonal antibodies having uniform properties produced by each of the clones are provided.

The hybridoma clones of the present invention are constructed by preparing a hybridoma of (a) myeloma cells, namely, malignant cells from early tumor of bone marrow and (b) antibody-producing cells from the spleens or lymph nodes of animals immunized with human BCDF, and by culturing and cloning this hybridoma and selecting the hybridoma as a clone capable of producing an antibody showing specificity to human BCDF.

The objective monoclonal antibody can be recovered from the culture supernatant obtained by culturing such a clone, by means of purification methods such as salting out, and ion exchange chromatography, etc. If necessary, the whole of supernatant can also be used. If it is intended to obtain the monoclonal antibody in large quantities, the anti-human BCDF antibody-producing hybridoma is intraperitoneally transplanted into a histocompatible animal, thymus-deficient nude mouse, etc. and proliferated, and the monoclonal antibody produced in the ascites of the animal is purified and recovered.

By the use of the anti-human BCDF monoclonal antibody provided by the present invention, it has become possible to determine the amount of human BCDF in blood, urine or body fluids such as synovia, etc. That is, human BCDF in blood, urine or body fluids can be readily assayed quantitatively by radioimmunoassay (RIA) or enzyme-linked immunoassay (EIA) using the anti-human BCDF antibody.

By performing radioimmunoassay (RIA) or enzyme-linked immunoassay (EIA) using the anti-human BCDF monoclonal antibody produced by the present invention, the present invention has opened a route for quantitatively determining a trace amount of human BCDF in blood, urine or body fluids with good sensitivity.

The radioimmunoassay (RIA) or enzyme-linked immunoassay (EIA) using the anti-human BCDF monoclonal antibody of the present invention may be performed by ordinary double antibody sandwich method.

In this case, it is preferred that the assay be performed with different kinds of a primary antibody and a secondary antibody, using two kinds of monoclonal antibodies, but a polyclonal antibody may also be used as a secondary antibody.

If the anti-human BCDF monoclonal antibody produced by the present invention is to be used for immunohistochemical staining, for example, an indirect method using the enzyme labelled antibody as described below may be applied.

After a topical tissue slice with inflammation or a smear specimen for cytological diagnosis has been fixed with alcohol and non-specific adsorption has been blocked with serum albumin, etc. an anti-human BCDF antibody is added to cause the reaction. After washing, peroxidase-labelled rabbit anti-mouse immunoglobulin (Ig) is reacted as a secondary antibody. After washing, a color forming reaction is effected with 3,3-diaminobentidine and hydrogen peroxide, whereby cells having intracellular human BCDF are stained to brown.

By performing binding of the anti-human BCDF monoclonal antibody produced by the present invention to a suitable support such as a carrier resin, etc. and subjecting to affinity chromatography, human BCDF contained in a medium used to culture cells or bacteria can be specifically purified thereby to easily obtain human BCDF having an extremely high purity. For example, the anti-human BCDF monoclonal antibody can be bound to a support such as Sepharose (manufactured by Pharmacia) activated with bromocyanide etc.; and by preparing a column with this monoclonal antibody-bound support and subjecting the culture medium to afffinity chromatography, human BCDF can easily be purified.

Further, the anti-human BDCF monoclonal antibody derived from MH166 clone orαBSF2-77 clone produced by the present invention can neutralize the human BCDF activity, as shown in the examples.

Therefore, by administering the human BCDF monoclonal antibody capable of neutralizing human BCDF activity to a patient suffering from an autoimmune disease such as a rheumatoid patient where human BCDF is excessively produced, the excessively produced human BCDF can be neutralized. Accordingly, the human BCDF monoclonal antibody of the present invention can be considered as a drug or treating autoimmune diseases such as rheumatoid arthritis.

It is also known that cancer cells such as myeloma or plasmacytoma are prolifereated by human BCDF. Therefore, the human BCDF antibody capable of neutralizing human BCDF activity may also be used as a drug for treating the above described cancers.

The antibody to be used may be either the monoclonal antibody itself or a F(ab') fragment obtained by removing the Fc portion by an enzyme. Further the antibody may also be a chimera antibody in which the Fc portion is replaced by human Fc.

In the composition for treating autoimmune diseases containing as an effective ingredient the human BDCF monoclonal antibody or the anti-cancer composition containing the human BCDF monoclonal antibody as an effective ingredient, it may be sufficent that the content of the human BCDF monoclonal antibody of the present invention be in the range of 0.001 to 100 wt%

Further the composition for treating autoimmune diseases or the anti-cancer composition containing as an effective ingredient the human BCDF monoclonal antibody may also contain a stabilizer such as serum albumin, an excipient such as mannitol, etc.

Furthermore, the composition for treating autoimmune diseases or the anti-cancer composition of the present invention may be used in combination with other chemotherapeutic agents.

For administering the composition for treating autoimmune diseases or the anti-cancer composition of the present invention, any conventional route, such as intravenous injection, intramuscular injection, subcutaneous injection, etc. may be applied.

It has been confirmed that the human BCDF monoclonal antibody of the present invention is safe.

As described above, utility over a wide range can be expected with respect to the anti-human BCDF monoclonal antibody of the present invention. Hereafter a method for the production of this anti-human BCDF monoclonal antibody is described.

The hybridoma is produced by fusing a myeloma cell and an antibody-producing cell. As the antibody-producing cell, spleen cells or lymph node cells from animals such as mouse or rat immunized with human BCDF may be used. The species of animal from which myeloma cells and antibody-producing cells are derived may be different as far as they are fusible but good results are generally obtained, using cells from the same species.

One preferred hybridoma for practicing the present invention is a hybridoma between a lymph node cell or spleen cell of a mouse immunized with human BDCF and mouse myeloma cell.

As will also be shown by the examples later described, for example, a hybridoma between an antibody-producing lymph node cell of a BALB/C mouse immunized with human BCDF emulsified with Freund's complete adjuvant and BALB/C mouse myeloma cell P3-X63-Ag8-U1 gave excellent results.

As the myeloma cell, there may be used, in addition to P3-X63-Ag8-U1, 8-azaguanine-resistant cell lines, such as P3-X63-Ag8, P3-NSI/1-Ag4-1, MPC11-45.6.TG.1.7, SP2/V-Ag14, X63-Ag88-6.5.3 (which are derived from mouse), 210.RCY.Agl.2.3 (rat), SKO-007, GH15006TG-A12 (which are of human origin), etc.

Further the antibody-producing cell may also be otained for example, as follows. First, an animal such as mouse, rat, etc. is immunized with human BCDF. The human BCDF used here may be any of a recombinant product produced in Escherichia coli, etc. and further human BCDF purified from culture supernatants of human tonsil mononuclear cells, human peripheral blood mononuclear cells or human tumor cells such as human T lymphoma, or those derived from artifically produced hybridoma, etc.

Next, lymph node cells are prepared from the immunized animals. For the method for production, reference is made to, for example, "MEN-EXI JIKKEN SOSAHO (Procedure for Immune Experiment), page A447 (edited by the Association of Immunology, Japan, 1975).

The production of hybridoma and subsequent selection of anti-human BCDF antibody-producing clone can be performed, for example, as follows. Using polyethylene glycol (PEG) or Sendai virus (HVJ), the antibody-producing cell is fused with myeloma cell. The resulting hybridoma grows in a medium containing hypoxanthine, aminopterine and thymidine (hereafter simply referred to as HAT medium). The antibody-producing cell and myeloma cell which failed to be fused are both killed in the medium and only the hybridoma proliferates from each clone. From the grown hybridoma, a clone capable of producing the anti-human BCDF antibody is selected. All of the hybridoma clones do not necessarily produce the antibody. Further the antibodies produced from the respective clones have different specificities and all of the clones do not necessarily produce the anti-human BCDF antibody. Therefore, a clone capable of producing the antibody showing specificity to human BCDF must be selected.

Selection of the anti-human BCDF antibody-producing clone may be carried out, for example, as follows. That is, ¹²⁵I-human BCDF is prepared by the Bolton-Hunter method or the lactoperoxidase method and ¹²⁵I-human BCDF binding ability in the culture supernatant in hybridoma clone is determined. A hybridoma having a high binding ability in the supernatant is selected as the anti-human BCDF monoclonal antibody-producing clone. Further, selection may also be effected by enzyme-linked immunosorbent assy (ELISA) using human BCDF and enzyme-labelled anti-mouse Ig antibody.
Examples of the thus obtained hybridoma clone include a cell called MH-60 (FERM BP-1971), a cell called MH-166 (FERM BP-1972), a cell called αBSF2-6 (FERM BP-1973), a cell called αBSF2-39 (FERM BP-1974) and a cell called αBSF2-77 (FERM BP-1975).

Next, the method for producing the monoclonal antibody in large quantities will be described. After MH-60 (FERM BP-1971), MH-166 (FERM BP-1972), αBSF2-6 (FERM BP-1973), αBSF2-39 (FERM BP-1974) and αBSF2-77 (FERM BP-1975) are subcultured consecutively over long periods of time or grown in histocompatible animal or thymus-deficient nude mouse, the monoclonal antibody may be recovered in a conventional manner.

The thus obtained monoclonal antibody has the following properties.
(1) Anti-human BCDF monoclonal antibody produced by MH-60 (FERM BP-1971)
   (a) kind of immunoglobulin : IgM
   (b) molecular weight : 900,000 daltons
   (c) molecular extinction coefficient : E280 nm = 14.0
   (d) It specifically binds to human BCDF.
(2) Anti-human BCDF monoclonal antibody produced by MH-166 (FERM BP-1972)
   (a) kind of immunoglobulin : IgG
   (b) molecular weight : 150,000 daltons
   (c) molecular extinction coefficient : E280 nm = 14.0
   (d) It specifically binds to human BCDF.
   (e) It neutralizes human BCDF activity.
(3) Anti-human BCDF monoclonal antibody produced by αBSF2-6 (FERM BP-1973)
   (a) kind of immunoglobulin : IgM
   (b) molecular weight : 900,000 daltons
   (c) molecular extinction coefficient : E280 nm = 14.0
   (d) It specifically binds to human BCDF.
(4) Anti-human BCDF monoclonal antibody produced by αBSF2-39 (FERM BP-1974)
   (a) kind of immunoglobulin : IgM
   (b) molecular weight : 900,000 daltons
   (c) molecular extinction coefficient : E280 nm = 14.0
   (d) It specifically binds to human BCDF.
(5) Anti-human BCDF monoclonal antibody produced by αBSF2-77 (FERM BP-1975)
   (a) kind of immunoglobulin : IgG
   (b) molecular weight : 150,000 daltons
   (c) molecular extinction coefficient : E280 nm = 14.0
   (d) It specifically binds to human BCDF.
   (e) It neutralizes human BCDF activity.

The anti-human BCDF monoclonal antibody of the present invention allows the measurement of the concentration of human BCDF in a sample such as blood, urine or body fluids, etc. containing a trace amount of human BCDF together with substances interfering the biological assay by radioimmunoassay (RIA) or enzyme-linked immunoassay (EIA).

It may also be used for immunohistochemical staining.

It is further possible to specifically purify human BCDF by affinity chromatography on a column prepared by binding the anti-human BCDF monoclonal antibody to a carrier resin.

Further the MH-166 (FERM BP-1972) - and αBSF2-77 (FERM BP-1975) - derived anti-human BCDF monoclonal antibody (IgG), can be used as a drug for treating a patient suffering from a disease in which BCDF is excessively produced and where BCDF is responsible for tumor growth.

The usefulness of the anti-human BCDF monoclonal antibody of in the present invention may be shown by the enclosed drawings, where
Figure 1 is a drawing showing that the concentration of human BCDF can be determined by ELISA using MH 166-derived anti-human BCDF monoclonal antibody.
Figure 2 is a drawing showing that the concentration of human BCDF can be determined by ELISA using MH 60-derived anti-human BCDF monoclonal antibody.
Figure 3 is a drawing showing that MH 166-derived anti-human BCDF monoclonal antibody can neutralize the activity of human BCDF.
Figure 4 is a drawing showing that αBSF2-77-derived anti-human BCDF monoclonal antibody can neutralize the activity of human BCDF.

Hereinafter the present invention will be described in more detail, with reference to the examples.

### Example 1

### Production of anti-human BCDF antibody-producing hybridoma

Human BCDF, 10 µg, produced in Escherichia coli and purified was intraperitoneally injected to BALB/C mice (female, 6 weeks old) together with an equal volume of Freund's complete adjuvant to effect immunization. After frequent immunization 4 to 6 times, the lymph node was ectomized and a cell suspension was prepared. The suspension was used as antibody-producing cells for fusion. On the other hand, P3X63-Ag8-U1 (P3U1) was used as myeloma cell. Both cells were fused by the polyethylene glycol methol.

The fused cells were suspended in HAT medium (Science, 145 , 709 (1964)) and 0 to 10 ng/ml of human BCDF was added to the suspension. The suspension was cultured in a 96 well plate.

With respect to wells in which hybridoma grew, a quantity of anti-human BCDF antibody in the culture supernatant was assayed by the method described in Example 2.

The cloning efficiency of hybridoma is shown in Table 1.

**Table 1**

| | Concentration of Human BCDF (ng/ml) | | |
|---|---|---|---|
| | 0 | 0.1 | 1 |
| Well in which hybridoma grew (%) | 14 | 26 | 31 |
| Well containing anti-human BCDF monoclonal antibody (%) out of the hybridoma growing wells | 0 | 35 | 70 |

### Example 2

### Assay for anti-human BCDF antibody-producing hybridoma

The hybridoma grown in HAT medium was transferred into 10% FCS-containing RPMI-1640 medium. The culture supernatant was used as a sample for assaying anti-human BCDF antibody as it was.

The assay was performed as follows. Goat anti-mouse Ig antibody (manufactured by Tago Co., Ltd.) was separately charged onto a 96 well flexible culture plate and allowed to stand at 4°C overnight to immobilize. The unbound goat anti-mouse Ig antibody was removed and 0.5% BSA-containing PBS was separately charged. The system was allowed to stand at room temperature for an hour. Next, the unbound BSA was removed and the hybridoma culture supernatant was separately charged in each well and allowed to settle for 2 hours. After washing, iodized human BCDF (¹²⁵I-human BCDF) prepared by the Bolton-Hunter method was added to the system and allowed to settle for an hour. Lastly, the system was washed with PBS. After drying, each well on the plate was cut out with a cutter and the amount of the bound human BCDF was determined by a γ-counter. Alternatively, human BCDF was fixed and, the aforesaid culture supernatant, alkali phosphatase-labelled anti-mouse Ig antibody and substrate were added in sequence to the system. An amount of the anti-human BCDF was measured at OD⁴⁰⁵ Among the thus obtained hybridomas, the culture supernatants are capable of specifically binding with human BCDF, with respect to 5 clones, MH-60 (FERM BP-1971), MH-166 (FERM BP-1972), αBSF2-6 (FERM BP-1973), αBSF2-39 (FERM BP-1974) and αBSF2-77 (FERM BP-1975). These are shown in Table 2.

**Table 2**

| Binding Ability of Hybridoma Supernatant with Human BCDF | | |
|---|---|---|
| | Amount of ¹²⁵I-Human BCDF Bound (c.p.m.) | Amount of Anti-Human BCDF Bound (OD₄₀₅) |
| Blank | 100 | 0.008 |
| MH 60 | 300 | N.D |
| MH 160 | 1,500 | 0.683 |
| α-BSF2-6 | N.D | 1.215 |
| α-BSF2-39 | N.D | 0.957 |
| α-BSF2-77 | N.D | 0.838 |
| N.D : not determined | | |

### Example 3

### ELISA using anti-human BCDF monoclonal antibody

Purified anti-human BCDF monoclonal antibody {10 µg/ml of MH-60 (FERM BP-1971)- derived IgM or 0.1 µl/ml MH-166 (FERM BP-1972)-derived IgG} was separately charged by 100 µl each onto a 96 well immunoplate (manufactured by Nunc Co., Ltd.) and allowed to stand at 4°C overnight. An excess amount of the unbound anti-human BCDF antibody was removed and in order to block 0.5% BSA-containing PBS was separately charged and allowed to stand at room temperature for an hour. Next, the unbound BSA was removed and, a test sample containing BCDF and standard human BCDF were separately charged in each well. The system was settled for 2 hours. After washing, 100 µl of 0.1 µg/ml affinity-purified rabbit anti-BCDF polyclonal antibody was added thereto. The system was settled for 2 hours. After washing, 5,000-fold diluted alkali phosphatase labelled goat anti-rabbit Ig (manufactured by Tago Co., Ltd.) was added followed by settlement for 2 hours. Lastly, the system was washed and alkali phosphatase substrate (manufactured by Sigma Co., Ltd.) was added. The mixture was incubated at 37°C for 30 minutes and absorbancy of each well was measured at 405 nm.

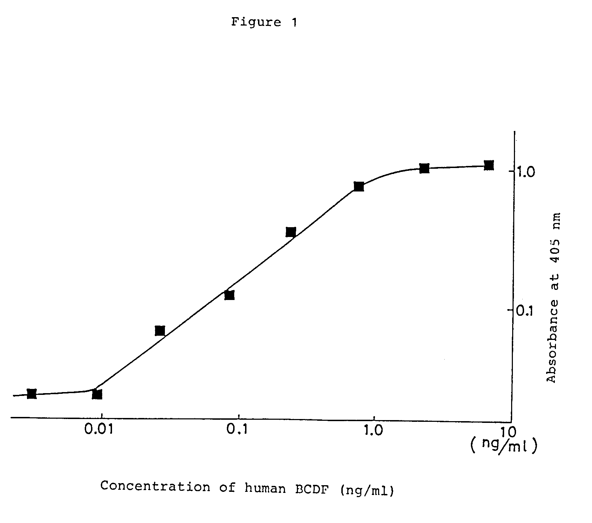

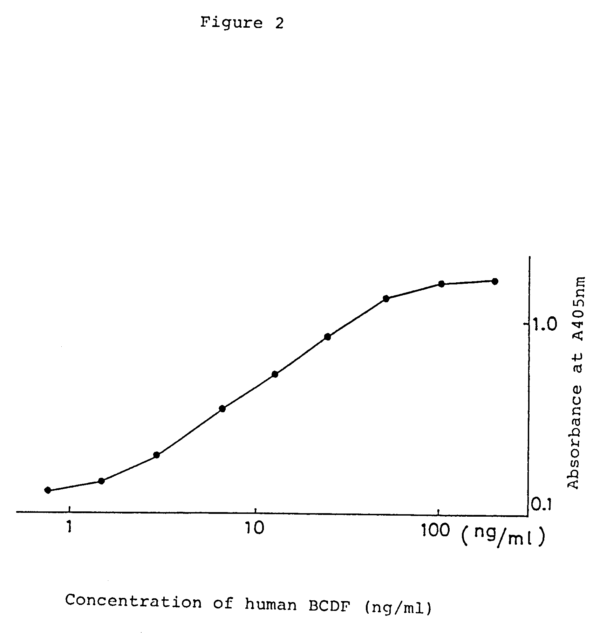

As a result of test using a known concentration of human BCDF, human BCDF in a range of 3 to 50 ng/ml and a range of 0.01 to 10 ng/ml was measurable with MH-60 (FERM BP-1971)-derived IgM and MH-166 (FERM BP-1972)-derived IgG, respectively, as shown in Figures 1 and 2.

### Example 4

### Inhibition of human BCDF activity by anti-human BCDF monoclonal antibody

(1) MH-60 (FERM BP-1971)-derived IgM and MH-166 (FERM BP-1972)-derived IgG (25 to 0 µg/ml) were independently added to 0.1 ng/ml of human BCDF followed by reacting at 37°C for 2 hours.

In order to examine the neutralizing activity of each anti-human BCDF monoclonal antibody, hybridoma MH 60 which grows dependent on human BCDF was added to each reaction solution in a cell density of 5 x 10⁴/ ml followed by culturing on a 96 well flat bottomed plate (Falcon 3072) for 42 hours.

1 µci/well of ³H-thymidine was added to the well. After incubation for further 6 hours, cells were harvested and inhibition of the human BCDF activity was determined by uptake of ³H-thymidine. That is, inhibition of the uptake of ³H-thymidine by MH60 cells depends on the concentration of anti-human BCDF monoclonal antibody.

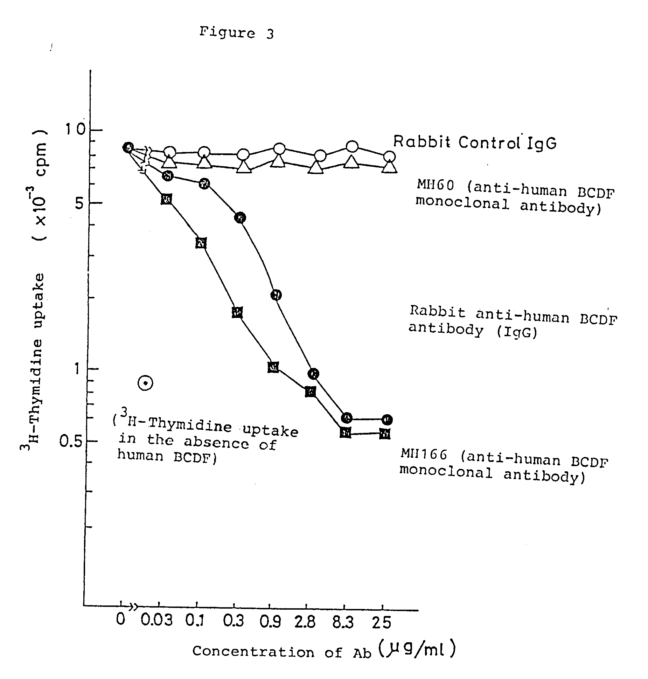

As shown in Figure 3, the MH 166-derived monoclonal antibody neutralized the human BCDF activity or inhibited augmented ³H-thymidine incorporation by the addition of BCDF specifically.
(2) αBSF2-77 (FERM BP-1975)-derived anti-human BCDF monoclonal antibody, 25 to 0 µg/ml, was added to 20 pg/ml of human BCDF followed by reacting at 37° for 2 hours.

In order to examine a neutralizing activity of this anti-human BCDF monoclonal antibody, hybridoma MH 60 which grows dependent on human BCDF was added to the reaction solution in cell density of 5 x 10⁴/ml followed by culturing on a 96 well flat bottomed plate (Falcon 3072) for 42 hours, in a manner similar to (1) described above.

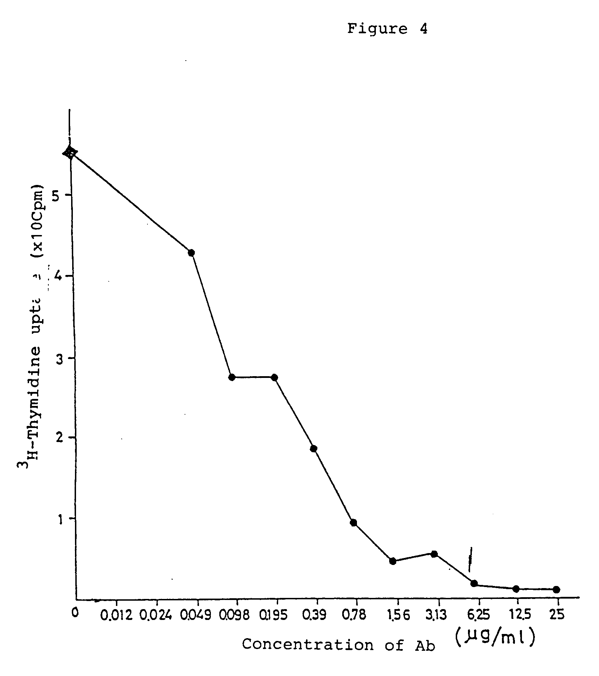

1 µci/well of ³H-thymidine was added to the well. After incubation for further 6 hours, cells were harvested and inhibition of the human BCDF activity was determined by uptake of ³H-thymidine. That is, inhibition of the uptake of ³H-thymidine by MH60 cells depends on a concentration of anti-human BCDF monoclonal antibody, as shown in Figure 4.

From the results of Examples (1) and (2), it was shown that αBSF2-77 (FERM BP-1975)- and MH-166 (FERM BP-1972)-derived monoclonal antibodies could be sufficiently used not only as an agent for treating cancer cells growing dependent upon human BCDF but also as an agent for treating patients suffering from autoimmune diseases such as rheumatoid patients in which human BCDF is excessively produced.

## Claims

1. A hybridoma capable of producing a monoclonal antibody specific for hBCDF, obtainable by immunising mice with hBCDF, isolating and immortalising the resulting lymphocytes, culturing the hybridomas thus obtained in the presence of hBCDF and selecting the hybridomas with hBCDF.

2. A hybridoma according to claim 1, which is selected from MH-60 (FERM BP-1971), MH-166 (FERM BP-1972), α-BSF2-6 (FERM BP-1973), α-BSF2-39 (FERM BP-1974) or α-BSF2-77 (FERM BP-1975).

3. A monoclonal antibody specific for hBCDF, obtainable from a hybridoma according to claim 1 or 2.

4. A treating agent for autoimmune diseases comprising a monoclonal antibody according to claim 3.

5. A treating agent for rheumatoid arthritis, comprising a monoclonal antibody according to claim 3.

6. An anti-cancer agent comprising a monoclonal antibody according to claim 3 as an effective ingredient.

7. A treating agent according to any of the claims 5 to 7, comprising the monoclonal antibody according to claim 3 in combination with other therapeutic agents.

8. Use of a monoclonal antibody according to claim 3 for detection of human B-cell differentiation factor.

## Patentansprüche

1. Hybridom, welches zur Produktion eines für humanen BCDF spezifischen, monoklonalen Antikörpers befähigt ist, erhältlich durch
Immunisieren von Mäusen mit humanem BCDF,
Isolieren und Immortalisieren der so erhaltenen Lymphozyten,
Züchten der so erhaltenen Hybridome in Anwesenheit von humanem BCDF und
Selektieren der Hybridome mit humanem BCDF.

2. Hybridom nach Anspruch 1, das ausgewählt ist unter MH-60 (FERM BP-1971), MH-166 (FERM BP-1972), α-BSF2-6 (FERM BP-1973), α-BSF2-39 (FERM BP-1974) oder α-BSF2-77 (FERM BP-1975).

3. Für humanes BCDF spezifischer monoklonaler Antikörper, erhältlich durch ein Hybridom nach Anspruch 1 oder 2.

4. Mittel zur Behandlung von Autoimmunkrankheiten, welches einen monoklonalen Antikörper nach Anspruch 3 enthält.

5. Mittel zur Behandlung von rheumatischer Arthritis, welches einen monoklonalen Antikörper nach Anspruch 3 enthält.

6. Antikrebsmittel, welches als Wirksubstanz einen monoklonalen Antikörper nach Anspruch 3 enthält.

7. Mittel zur Behandlung nach einem der Ansprüche 5 bis 7, welches den monoklonalen Antikörper nach Anspruch 3 in Kombination mit anderen therapeutischen Mitteln enthält.

8. Verwendung eines monoklonalen Antikörpers nach Anspruch 3 zum Nachweis von humanem B-Zell-Differenzierungsfaktor.

## Revendications

1. Hybridome capable de produire un anticorps monoclonal spécifique anti-hBCDF, pouvant être obtenu par immunisation de souris avec le hBCDF, isolement et immortalisation des lymphocytes obtenus, culture des hybridomes ainsi obtenus en présence de hBCDF et sélection des hybridomes à l'aide du hBCDF.

2. Hybridome selon la revendication 1, qui est choisi parmi MH-60 (FERM BP-1971), MH-166 (FERM BP-1972), αBSF2-6 (FERM BP-1973), αBSF2-39 (FERM BP-1974) et αBSF2-77 (FERM BP-1975).

3. Anticorps monoclonal spécifique anti-hBCDF, pouvant être obtenu à partir d'un hybridome selon la revendication 1 ou 2.

4. Agent de traitement pour des maladies auto-immunes, comprenant un anticorps monoclonal selon la revendication 3.

5. Agent de traitement pour la polyarthrite rhumatoïde, comprenant un anticorps monoclonal selon la revendication 3.

6. Agent anticancéreux comprenant un anticorps monoclonal selon la revendication 3 comme ingrédient actif.

7. Agent de traitement selon l'une quelconque des revendications 5 à 7, comprenant l'anticorps monoclonal selon la revendication 3 en combinaison avec d'autres agents thérapeutiques.

8. Utilisation d'un anticorps monoclonal selon la revendication 3 pour le décèlement du facteur de différenciation des lymphocytes B humain.
